# EUROPÄISCHE PATENTSCHRIFT

(11) **EP 3 262 091 B1**
(45) Veröffentlichungstag und Bekanntmachung des Hinweises auf die Patenterteilung: **25.12.2019**
(21) Anmeldenummer: 16706609.1
(22) Anmeldetag: 25.02.2016
(51) Int. Cl.: C08G 18/76, C07C 267/00, C08G 18/79, C08G 18/02, C08G 18/16

(54) **VERFAHREN ZUR HERSTELLUNG EINER POLYCARBODIIMIDE UMFASSENDEN ZUSAMMENSETZUNG MIT VERBESSERTER LAGERSTABILITÄT**
METHOD FOR PRODUCING A COMPOSITION COMPRISING POLYCARBODIIMIDES WITH IMPROVED STORAGE STABILITY
PROCÉDÉ DE FABRICATION D'UNE COMPOSITION COMPRENANT UN POLYCARBODIIMIDE PRÉSENTANT UNE STABILITÉ AU STOCKAGE AMÉLIORÉE

(30) Priorität: 26.02.2015 EP 15156661
(43) Veröffentlichungstag der Anmeldung: 03.01.2018
(73) Patentinhaber: Covestro Deutschland AG, 51373 Leverkusen (DE)
(72) Erfinder: ARNTZ, Hans-Detlef, 51491 Overath (DE); WERSHOFEN, Stefan, 41065 Mönchengladbach (DE); FELSKE, Ernst, 41464 Neuss (DE)
(74) Vertreter: Levpat
(86) Internationale Anmeldenummer: PCT/EP2016/054010
(87) Internationale Veröffentlichungsnummer: WO 2016/135256

(56) Entgegenhaltungen:
- EP-A1- 2 371 873
- EP-B1- 1 671 988

## Beschreibung

Die vorliegende Erfindung betrifft ein Verfahren zur Herstellung einer Polycarbodiimide umfassenden Zusammensetzung, umfassend den Schritt der Umsetzung einer Reaktionsmischung, welche ein aromatisches Polyisocyanat und einen Carbodiimidisierungs-Katalysator umfasst.

Bei der katalytisch unterstützten Herstellung von Carbodiimiden aus aromatischen Polyisocyanaten und insbesondere aus 4,4'-Diisocyanatodiphenylmethan (4,4'-MDI) kann die Reaktivität des Systems starken Schwankungen unterliegen. Dieses kann zumindest teilweise auf den Gehalt an hydrolysierbarem Chlor (HC) im System zurückgeführt werden. Diese Reaktivitätsschwankungen werden häufig durch eine Anpassung der Katalysatorkonzentration ausgeglichen, wobei als Katalysator in vielen Fällen ein Phospholinoxid zum Einsatz kommt. Eine erhöhte Phospholinoxidkonzentration beeinflusst zwar die Reaktionsgeschwindigkeit im erwünschten Sinne, führt jedoch zu einer deutlichen Abnahme der Produktstabilität. Die Abnahme der Produktstabilität offenbart sich durch eine Zunahme der Viskosität der Polycarbodiimid-haltigen Zusammensetzung.

Es hat in der Vergangenheit nicht an Versuchen gefehlt, den Gehalt an hydrolysierbarem Chlor in den Polyisocyanaten zu verringern. US 3,516,950 beschäftigt sich mit der Herstellung von Polyisocyanurat-Hartschäumen und führt in diesem Zusammenhang aus, dass es vorteilhaft sei, organische Polyisocyanate einzusetzen, welche keinen exzessiv hohen Gehalt an hydrolysierbarem Chlor aufweisen. Um den HC-Gehalt der Roh-Polyisocyanate zu verringern, können diese mit Kalk oder anderen schwach basischen Materialien behandelt werden. Alternativ können sie auf Temperaturen zwischen 150 °C und 220 °C erhitzt werden, während durch die Flüssigkeit ein Strom von Inertgas geleitet wird, um das Entfernen von Chlorwasserstoff zu unterstützen. In einem Beispiel wird ferner beschrieben, wie Roh-MDI im Stickstoffstrom für 20 Stunden auf 180 °C bis 190 °C erhitzt wird. Es wird berichtet, dass der NCO-Gehalt hierbei von 86.4% auf 80.0% des theoretischen NCO-Gehalts von MDI reduziert wird und der Gehalt an hydrolysierbarem Chlor von 0.4% auf 0.17% sinkt.

US 4,088,665 betrifft die partielle Carbodiimidisierung von Mono-, Di- und/oder Polyisocyanaten in Gegenwart einer dort spezifizierten phosphororganischen Verbindung, gefolgt vom Abbrechen der Carbodiimidisierungsreaktion durch Zugabe eines Carbamoylchlorids oder -bromids. In einem Beispiel wird beschrieben, wie ein Reaktionsgefäß mit 4,4'-MDI befüllt und mit Stickstoff gespült wird. Nach dem Spülen wird auf 65 °C für eine Stunde erhitzt.

Ein allgemeiner Überblick über die Herstellung lagerstabiler Carbodiimide findet sich in DE 10 2004 033 849 A1. Diese Patentanmeldung offenbart Verfahren zur Herstellung Carbodiimid- und/oder Uretonimingruppen aufweisender organischer Isocyanate durch teilweise Carbodiimidisierung von Isocyanatgruppen mit Katalysatoren vom Phospholin-Typ, und anschließendes Abstoppen der Carbodiimidisierungsreaktion durch den Zusatz einer silylierten Säure der Formel X-[Si(CH₃)₃]ₙ, wobei in der Formel X für den neutralen Säurerest steht, wie er durch Entfernen der aciden Wasserstoffatome aus einer n- basischen Säure mit einem pKₐ-Wert von max. 3 erhalten wird, wobei Halogenwasserstoffsäuren ausgenommen sind, und n eine ganze Zahl von 1 - 3 bedeutet. Zusätzlich zu der silylierten Säure wird eine nicht silylierte Säure und/oder ein Säurechlorid und/oder ein Sulfonsäureester zugesetzt.

US 2005/0282993 A1 beschreibt ein Verfahren zur Herstellung flüssiger lagerstabiler organischer Isocyanate mit Carbodiimid- und/oder Uretonimingruppen. Hierbei werden saure Verunreinigungen in dem organischen Isocyanat mit einem Säurefänger neutralisiert. Anschließend erfolgt eine partielle Carbodiimidisierung in Gegenwart eines Katalysators vom Phosphoroxid-Typ, gefolgt vom Abbrechen der Carbodiimidisierungsreaktion durch Zugabe einer Säure.

EP 1 971 623 B1 hat sich die Aufgabe gestellt, ein einfaches und wirtschaftliches Verfahren zur Herstellung flüssiger, lagerstabiler und heller Carbodiimid- und/oder Uretonimingruppen aufweisender Isocyanatmischungen zur Verfügung zu stellen. Die Patentschrift offenbart ein Verfahren zur Herstellung Carbodiimid- und/oder Uretonimingruppen aufweisender organischer Isocyanate, bei dem ein oder mehrere organische Isocyanate mit einer Hazen-Farbzahl von < 100 APHA, bevorzugt < 50 APHA, mit Katalysatoren vom Phospholin-Typ teilweise carbodiimidisiert werden, und anschließend die Carbodiimidisierungsreaktion abgestoppt wird, wobei die Carbodiimidisierung in Gegenwart eines silylierten Säureamids durchgeführt wird.

Als Vorteile werden angegeben, dass durch die Anwesenheit eines silylierten Säureamids während der Carbodiimidisierung die Reaktivität des Reaktionsgemisches erhöht und/oder vereinheitlicht würde. Dadurch könne die erforderliche Reaktionszeit erniedrigt werden bzw. niedrig gehalten werden und/oder die erforderliche Katalysatormenge reduziert werden. Sowohl die Carbodiimid- und/oder Uretonimingruppen-haltigen Isocyanate als auch die daraus hergestellten Prepolymere weisen gemäß der Aussage dieser Patentschrift eine gute Lagerstabilität und eine helle Farbe auf. Diskutiert wird auch anhand der Vergleichsbeispiele 1 und 2, welchen Einfluss der erhöhte Gehalt an hydrolysierbarem Chlor auf die Reaktivität bzw. die Reaktionszeit hat. In den patentgemäßen Beispielen 1 und 2 wird eine gegenüber dem Vergleichsbeispiel 2 eine verbesserte Reaktivität erreicht, die bei gleicher Konzentration an eingesetztem Katalysator zu kürzeren Reaktionszeiten in den patentgemäßen Beispielen 1 und 2 führt.

EP 2 371 873 A1 bezieht sich auf die Herstellung von Polycarbodiimid und von wässrigen Dispersionen von Polycarbodiimid und beschreibt ein Verfahren zur Herstellung einer Polycarbodiimide umfassenden Zusammensetzung, umfassend den Schritt der Umsetzung einer Reaktionsmischung, welche ein aliphatisches oder cylcoaliphatisches Polyisocyanat und einen Carbodiimidisierungs-Katalysator umfasst, dadurch gekennzeichnet, dass während der Umsetzung in Gegenwart von 50 - 3000 ppm Carbodiimidiserungskatalysator die Reaktionsmischung bei einer Temperatur von 160 °C bis 230 °C gehalten wird. Das Anlegen von Vakuum (500 mbar in den Beispielen) oder das Einleiten von Stickstoff oder eine Kombination beider Merkmale wird als vorteilhaft dargestellt, indem hierdurch die Reaktionsgase entfernt werden und so die Carbodiimidisierungsreaktion beschleunigt wird (Absatz [0011], Beispiele). Auch wenn auf die deutlich milderen Reaktionsbedingungen für die Umsetzung *aromatischer* Isocyanate hingewiesen wird, war nicht zu erwarten, dass *die Lagerstabilität* von teilcarbodiimidisierten aromatischen Polyisocyanaten durch die Anwendung einer solchen Maßnahme auf das Polyisocyanat vor und/oder während dessen Umsetzung verbessert werden kann.

In EP 1 671 988 B1 wird u. a. die Durchführung der Carbodiimidisierungsreaktion unter Stickstoff beschrieben, worunter der Fachmann versteht, dass der Gasraum über der flüssigen Reaktionsmischung mit Stickstoff ausgefüllt war, was nicht gleichbedeutend ist mit einem *Durchleiten* eines Stickstoffstroms durch das Polyisocyanat vor und/oder während dessen Umsetzung.

WO 2007/076998 A1 offenbart ein Verfahren zur Herstellung Carbodiimid- und/oder Uretonimingruppen aufweisender organischer Isocyanate, bei dem ein oder mehrere organische Isocyanate mit einer Hazen-Farbzahl von ≤ 100 APHA, bevorzugt ≤ 50 APHA, mit Katalysatoren vom Phospholin-Typ teilweise carbodiimidisiert werden, und anschließend die Carbodiimidisierungsreaktion abgestoppt wird. Die Carbodiimidisierung wird in Gegenwart mindestens eines sekundären oder tertiären Amins, welches wenigstens einen aliphatischen oder cycloaliphatischen oder araliphatischen, ggf. substituierten und ggf. Heteroatome enthaltenden, Kohlenwasserstoffrest enthält, durchgeführt. Gemäß dieser Patentanmeldung wird durch den Einsatz eines sekundären oder tertiären Amins, welches wenigstens einen aliphatischen oder cycloaliphatischen oder araliphatischen, ggf. substituierten und ggf. Heteroatome enthaltenden, Kohlenwasserstoffrest enthält, erreicht, dass die Reaktivität des Ausgangsisocyanats erhöht wird. Dies könne z.B. ursächlich dadurch erfolgen, dass sie der reaktivitätsvermindernden Wirkung von potentiellem HCl abspaltenden Nebenkomponenten im Ausgangsisocyanat entgegenwirken, indem sie als Basen wirken und HCl als Hydrochlorid binden. Aber auch andere Wirkmechanismen seien möglich.

Als Vorteile des beschriebenen Verfahrens werden angeführt, dass durch die Anwesenheit eines sekundären oder tertiären Amins, welches wenigstens einen aliphatischen oder cycloaliphatischen oder araliphatischen, ggf. substituierten und ggf. Heteroatome enthaltenden, Kohlenwasserstoffrest enthält, während der Carbodiimidisierung die Reaktivität des Reaktionsgemisches erhöht und/oder vereinheitlicht würden. Dadurch könne die erforderliche Reaktionszeit erniedrigt werden bzw. niedrig gehalten werden und/oder die erforderliche Katalysatormenge reduziert werden. Sowohl die Carbodiimid- und/oder Uretonimingruppen-haltigen Isocyanate als auch die daraus hergestellten Prepolymere weisen laut Aussage dieser Patentanmeldung zudem eine gute Lagerstabilität und eine helle Farbe auf.

Die vorliegende Erfindung hat sich die Aufgabe gestellt, ein Verfahren zur Herstellung von Polycarbodiimid-Zusammensetzungen bereitzustellen, welches mit geringeren Mengen an im Produkt verbleibenden Einsatzstoffen auskommt und wobei die erhaltenen Polycarbodiimide dennoch lagerstabil (im Sinne einer geringeren Viskositätszunahme) sind.

Erfindungsgemäß gelöst wird die Aufgabe durch ein Verfahren zur Herstellung einer Polycarbodiimide umfassenden Zusammensetzung, umfassend den Schritt der Umsetzung einer Reaktionsmischung, welche ein Polyisocyanat und einen Carbodiimidisierungs-Katalysator umfasst, wobei vor der Umsetzung das Polyisocyanat bei einer Temperatur von ≥ 80 °C bis ≤ 150 °C und einem Druck von ≥ 1 mbar bis ≤ 500 mbar mittels Durchleiten eines Inertgases behandelt wird und/oder während der Umsetzung die Reaktionsmischung bei einer Temperatur von ≥ 80 °C bis ≤ 150 °C und einem Druck von ≥ 1 mbar bis ≤ 500 mbar mittels Durchleiten eines Inertgases behandelt wird, wobei weiterhin in der Reaktionsmischung der Gehalt an hydrolysierbarem Chlor ≤ 10 ppm beträgt.

Es wurde gefunden, dass durch eine Konditionierung des Polyisocyanats vor oder während seines Einsatzes in der Herstellung der Polycarbodiimide der Gehalt an hydrolysierbarem Chlor im Reaktionssystem verringert werden kann. Ohne auf eine Theorie festgelegt zu sein wird angenommen, dass die angestrebte erhöhte Lagerstabilität durch einen verringerten Gehalt des im Endprodukt verbleibenden Katalysators erreicht wird. Dass geringere Katalysatormengen überhaupt eingesetzt werden können, hängt wiederum mit der geringeren Menge an Säure, ausgedrückt als Gehalt an hydrolysierbarem Chlor, im System zusammen.

Die durch das erfindungsgemäße Verfahren erhaltenen Produkte können neben Carbodiimidgruppen auch Uretonimingruppen enthalten. Der Gehalt an freien NCO-Gruppen kann vorzugsweise in einem Bereich von ≥ 27 Gewichts-% bis ≤ 33 Gewichts-% liegen.

Für das erfindungsgemäße Verfahren geeignet sind aromatische Polyisocyanate, wobei in den Begriff "Polyisocyanate" auch Diisocyanate mit eingeschlossen sind.

Geeignet sind insbesondere aromatische Di- und polyisocyanate wie Toluylendiisocyanat und Di- und Polyisocyanate der Diphenylmethanreihe. Zu nennen sind insbesondere:
- Aromatische Diisocyanate wie 2,4- und/oder 2,6-Diisocyanatotoluol (TDI), 2,2'-, 2,4'- und/oder 4,4'-Diisocyanatodiphenylmethan (MDI) bzw. beliebige Gemische derartiger aromatischer Diisocyanate,
- Di- und Polyisocyanatgemische der Diphenylmethanreihe mit einem Gehalt an monomeren Diisocyanatodiphenylmethan-Isomeren von 80 bis 100 Gew.-% und einem Gehalt an höher als difunktionellen Polyisocyanaten der Diphenylmethanreihe von 0 bis 20 Gew.-%, wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 Gew.-% ergänzen.

Als Katalysatoren kommen für das erfindungsgemäße Verfahren insbesondere organische Phosphoroxide in Betracht.

Die Temperaturen für die Behandlung des Polyisocyanats beziehungsweise der Reaktionsmischung betragen erfindungsgemäß jeweils ≥ 80 °C bis ≤ 150 °C. Ein bevorzugter Temperaturbereich ist jeweils und unabhängig voneinander ≥ 85 °C bis ≤ 120 °C, mehr bevorzugt ≥ 90 °C bis ≤ 100 °C.

Die Drücke für die Behandlung des Polyisocyanats beziehungsweise der Reaktionsmischung betragen erfindungsgemäß jeweils ≥ 1 mbar bis ≤ 500 mbar. Ein bevorzugter Druckbereich ist jeweils und unabhängig voneinander ≥ 1 mbar bis ≤ 300 mbar, mehr bevorzugt ≥ 1 mbar bis ≤ 100 mbar.

Durch die vergleichsweise geringen Temperaturen während der Behandlung erfolgt selbst bei einem Druck von 1 mbar keine nennenswerte Destillation des Polyisocyanats. Ferner werden unerwünschte thermische Nebenreaktionen des Polyisocyanats vermieden. Somit lässt sich das erfindungsgemäß vorbehandelte Polyisocyanat ohne weitere Aufarbeitung oder Aufreinigung in einer Carbodiimidisierungsreaktion einsetzen. Das Gleiche gilt, wenn die Reaktionsmischung währen der Reaktion zum Carbodiimid erfindungsgemäß behandelt wird.

Wenn der Weg gewählt wird, dass das Polyisocyanat nur vor Beginn der Carbodiimidisierungsreaktion erfindungsgemäß behandelt wird, kann die eigentliche Carbodiimidierungsreaktion selbstverständlich unter den für diese Reaktion üblichen Bedingungen wie z.B. angegeben in EP 1 671 988 B1 oder EP 1 820 796 B1 durchgeführt werden. Die Carbodiimidisierungsreaktion kann sowohl im Vakuum, bei Atmosphärendruck oder bei leichtem Überdruck durchgeführt werden.

Das dritte Element der erfindungsgemäßen Behandlung ist neben dem bestimmten Temperaturbereich und dem bestimmten Druckbereich ferner, dass währenddessen ein Inertgas durch das Polyisocyanat oder durch die Reaktionsmischung geleitet wird. Als Inertgase kommen insbesondere Edelgase wie Argon und weiterhin Stickstoff in Frage. Es wird angenommen, dass das Inertgas gasförmigen Chlorwasserstoff abtransportieren kann, wodurch es zu einer Abnahme des Gehalts an hydrolysierbarem Chlor im Polyisocyanat oder der Reaktionsmischung kommt.

Das Durchleiten des Inertgases kann beispielsweise mittels eines Gaseintragrührers oder mittels eines Siebbodens in einem Reaktionsbehältnis erreicht werden.

Es ist im erfindungsgemäßen Verfahren weiterhin vorgesehen, dass in der Reaktionsmischung der Gehalt an hydrolysierbarem Chlor ("HC-Gehalt") ≤ 10 ppm beträgt. Vorzugsweise beträgt der Gehalt ≥ 0.1 ppm bis ≤ 10 ppm, mehr bevorzugt ≥ 1 ppm bis ≤ 5 ppm. Dieses kann durch die zuvor beschriebene Behandlung des Polyisocyanats erfolgen.

Die Bestimmung des Gehalts an hydrolysierbarem Chlor in Isocyanaten erfolgt durch Urethanisierung, Hydrolyse und potentiometrische Titration mit Silbernitrat an einer Silber/Silberchlorid-Einstabmesskette. Beispielsweise wird im Fall des MDI der HC-Wert durch Reaktion von MDI mit niederen Alkoholen wie Methanol, ermittelt (siehe ASTM D5523-94 für monomeres MDI oder ASTM5629-99 beziehungsweise 6099-03 für polymeres MDI).

Spezielle Ausführungsformen der vorliegenden Erfindung werden nachfolgend geschildert. Sie können beliebig kombiniert werden, sofern sich aus dem Kontext nicht eindeutig das Gegenteil ergibt.

In einer Ausführungsform des erfindungsgemäßen Verfahrens beträgt in der Reaktionsmischung der Gehalt des Carbodiimidisierungs-Katalysators ≤ 5 ppm, bevorzugt < 3,5 ppm, besonders bevorzugt < 3 ppm. Der Gehalt ist hierbei als Gewichtsanteil des Katalysators, bezogen auf das Gesamtgewicht der Reaktionsmischung, zu verstehen. Solch niedrige Katalysatorgehalte sind wegen des niedrigen HC-Gehalts in der Reaktionsmischung möglich. Dementsprechend ist auch der Gehalt an im Endprodukt verbleibendem Katalysator, welcher während der Lagerung unerwünschte Nebenreaktionen katalysiert, geringer. Dann steigt die Lagerstabilität. Bevorzugt ist ein Katalysatorgehalt von ≥ 0.1 ppm bis ≤ 2.5 ppm, mehr bevorzugt von ≥ 0.5 ppm bis ≤ 1.5 ppm.

In einer weiteren Ausführungsform des erfindungsgemäßen Verfahrens ist das Polyisocyanat Diphenylmethandiisocyanat. Bevorzugt ist hierbei 4,4'-MDI.

In einer weiteren Ausführungsform des erfindungsgemäßen Verfahrens ist der Carbodiimidisierungs-Katalysator ein Phospholinoxid. Solche Katalysatoren sind beispielsweise aus EP 515 933 A1 und US 6,120,699 bekannt. Typische Beispiele dieser Katalysatoren sind:

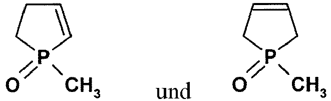

Weitere Beispiele sind 1-Phenyl-1-oxo-1-phosphacyclopent-2-en und 1-Phenyl-1-oxo-1-phosphacyclopent-3-en. Geeignet sind auch ein technisches Gemisch aus 1-Methyl-1-oxo-1-phosphacyclopent-2-en und 1-Methyl-1-oxo-1-phosphacyclopent-3-en oder ein technisches Gemisch aus 1-Phenyl-1-oxo-1-phosphacyclopent-2-en und 1-Phenyl-1-oxo-1-phosphacyclopent-3-en.

In einer weiteren Ausführungsform des erfindungsgemäßen Verfahrens erfolgt die Umsetzung in Abwesenheit eines Säurefängers. Durch den niedrigen HC-Gehalt ist ein Säurefänger nicht erforderlich. Ausgeschlossene Säurefänger sind insbesondere Basen wie Kalk oder Epoxide.

In einer weiteren Ausführungsform des erfindungsgemäßen Verfahrens wird nach dem Ende der Umsetzung der Reaktionsmischung zu dem hierdurch erhaltenen Produkt ein Stopper hinzugefügt. Dieser Stopper desaktiviert den Katalysator. Als Stopper geeignete Verbindungen sind z.B. Trifluormethansulfonsäureester, Trimethylsilyltriflat sowie andere auf Kohlenstoff basierende oder anorganische Säuren zu nennen. Vorzugsweise wird dieser Stopper im Zusammenhang mit einem Phospholinoxid-Katalysator eingesetzt. Ein bevorzugter Stopper ist Ethyltriflat.

Vorzugsweise wird der Trifluormethansulfonsäureester in einer Menge von ≥ 10 ppm bis ≤ 100 ppm hinzugefügt, mehr bevorzugt von ≥ 15 ppm bis ≤ 50 ppm. Der Gehalt ist hierbei als Gewichtsanteil des Stoppers, bezogen auf das Gesamtgewicht der Reaktionsmischung, zu verstehen.

In einer weiteren Ausführungsform des erfindungsgemäßen Verfahrens wird nach dem Ende der Umsetzung der Reaktionsmischung zu dem hierdurch erhaltenen Produkt kein Säurefänger hinzugefügt. Durch den niedrigen HC-Gehalt ist ein Säurefänger auch im Endprodukt nicht erforderlich. Ausgeschlossene Säurefänger sind insbesondere Basen wie Kalk oder Epoxide.

In einer weiteren Ausführungsform des erfindungsgemäßen Verfahrens wird nach dem Ende der Umsetzung der Reaktionsmischung zu dem hierdurch erhaltenen Produkt ein Polyisocyanat hinzugefügt. Auf diese Weise können Carbodiimid-haltige Isocyanatblends hergestellt werden. Hinzugefügte Isocyanate können gleich oder verschieden von dem oder den zur Carbodiimidisierung eingesetzten aromatischen Polyisocyanaten sein. Als Vertreter der aliphatischen und/oder cycloaliphatischen Diisocyanate sind beispielhaft zu nennen Isophorondiisocyanat, Hexamethylendiisocyanat und Dicyclohexylmethandiisocyanat (jeweils die reinen Isomeren sowie beliebige Isomerengemische). Als Vertreter der araliphatischen Diisocyanate sind beispielhaft zu nennen die verschiedenen Isomere der Xylidendiisocyanate.

Geeignet sind insbesondere aromatische Di- und polyisocyanate wie Toluylendiisocyanat und Di- und Polyisocyanate der Diphenylmethanreihe. Zu nennen sind insbesondere aromatische Diisocyanate wie 2,4- und/oder 2,6-Diisocyanatotoluol (TDI), 2,2'-, 2,4'- und/oder 4,4'-Diisocyanatodiphenylmethan (MDI) bzw. beliebige Gemische derartiger aromatischer Diisocyanate; weiterhin Di- und Polyisocyanatgemische der Diphenylmethanreihe mit einem Gehalt an monomeren Diisocyanatodiphenylmethan-Isomeren von 80 bis 100 Gew.-% und einem Gehalt an höher als difunktionellen Polyisocyanaten der Diphenylmethanreihe von 0 bis 20 Gew.-%, wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 Gew.-% ergänzen.

In einer weiteren Ausführungsform des erfindungsgemäßen Verfahrens wird nach dem Ende der Umsetzung der Reaktionsmischung das hierdurch erhaltene Produkt mit einem Polyol zur Reaktion gebracht. So können Carbodiimid-haltige Präpolymere erhalten werden. Geeignete Polyole sind sowohl einfache mehrwertige Alkohole des Molekulargewichtsbereiches 62 bis 599 g/mol, vorzugsweise 62 bis 300 g/mol, wie z.B. Ethylenglykol, Trimethylolpropan, Propandiol-1,2, Butandiol-1,2 oder Butandiol-2,3, Hexandiol, Octandiol, Dodecandiol und/oder Octadecandiol, insbesondere jedoch höhermolekulare Polyetherpolyole und/oder Polyesterpolyole der aus der Polyurethanchemie an sich bekannten Art mit Molekulargewichten von 600 bis 8000 g/mol, vorzugsweise 800 bis 4000 g/mol, die mindestens zwei, in der Regel 2 bis 8, vorzugsweise 2 bis 4 primäre und/oder sekundäre Hydroxylgruppen aufweisen.

### Beispiele

Die vorliegende Erfindung wird durch die nachfolgenden Beispiele näher erläutert, ohne jedoch darauf beschränkt zu sein. Die in den Beispielen verwendeten Begriffe bedeuten:
44M: 4,4'-Diphenylmethandiisocyanat (NCO-Gehalt: 33,6 Gewichts-%)
PHO: Phospholinoxid (Carbodiimidisierungs-Katalysator); technisches Gemisch aus 1-Methyl-1-oxo-1-5 phosphacyclopent-2-en und 1-Methyl-1-oxo-1-phosphacyclopent-3-en, 1 gewichtsprozentige Lösung in Toluol
ETF: Ethyltriflat (Stopper); Trifluormethansulfonsäureethylester

Zur Bestimmung des Gehalts an hydrolysierbarem Chlor (HC-Wert) wurde die Isocyanatprobe mit Methanol versetzt und 10 Minuten unter Rückfluss urethanisiert. Anschließend wurde die Mischung nach Verdünnen mit Wasser durch Kochen unter Rückfluss hydrolysiert. Das hierbei gebildete ionogene Chlor wurde nach Ansäuern mit Salpetersäure und Aufstockung mit einer bekannten Masse Natriumchlorid argentometrisch mit einer Silbernitratmaßlösung titriert. Die Titration wurde mit inkrementeller Reagenzdosierung und automatischer Äquivalenzpunkt-Auswertung driftkontrolliert (Gleichgewichtstitration) durchgeführt. Aus der Einwaage der Isocyanatprobe und dem Verbrauch an Silbernitratmaßlösung wurde der Gehalt an hydrolysierbarem Chlor unter Berücksichtigung der Aufstockung errechnet.

Die Viskosität wurde mit Hilfe des Rheometers Physika MCR 51 der Fa. Anton Paar, Ostfildern, DE mit einer Scherrate von 500 - 5000 l/s bei 25 °C bestimmt.

Der NCO-Gehalt des Reaktionsprodukts wurde nach DIN EN 1242 bestimmt.

### Beispiel 1: Effekt verschiedener Behandlungen von 44M auf den HC-Wert

Proben der gleichen Charge von 44M wurden den in der nachfolgenden Tabelle angegebenen Bedingungen unterworfen:

| Beispiel Nr. | 1-1 (Vergleich) | 1-2 (Vergleich) | 1-3 (erfindungsgemäß) |
|---|---|---|---|
| HC-Wert vor Behandlung | 17 ppm | 17 ppm | 17 ppm |
| Behandlung | Keine | 100 °C, 1 mbar Vakuum, 2 Stunden Dauer | 100 °C, 1 mbar Vakuum, Durchleiten von Stickstoff, 2 Stunden Dauer |
| HC-Wert nach Behandlung | 17 ppm | 11 ppm | 5 ppm |

### Beispiel 2: Herstellung von Carbodiimid-Zusammensetzungen

### Allgemeine Arbeitsvorschrift:

10 kg technisches 44M, das 750 ppm 3,5-Di-tert-butyl-4-hydroxytoluol enthielt, wurden unter N₂/Rühren auf ca. 90 °C erhitzt. Anschließend wurde die in der nachfolgenden Tabelle angegebene Menge Katalysator hinzugegeben. Das Reaktionsgemisch wurde unter N₂/Rühren bis zum Erreichen des erwünschten NCO-Gehaltes auf ca. 95 °C erhitzt. Danach wurde die Carbodiimidisierung durch Zugabe des Stoppers ETF beendet abgestoppt und es wurde 1 Stunde nachgerührt.

Zum Zweck der besseren Vergleichbarkeit wurde das Carbodiimid mit einer so angepassten PHO-Konzentration hergestellt, dass die Reaktionen sehr ähnliche Geschwindigkeitsverläufe aufwiesen und nach 4.5 Stunden beendet werden konnten.

| Beispiel Nr. | 2-1 (Vergleich) | 2-2 (Vergleich) | 2-3 (erfindungsgemäß) |
|---|---|---|---|
| 44M-Probe aus Beispiel | 1-1 | 1-2 | 1-3 |
| HC-Wert des 44M | 17 ppm | 17 ppm | 5 ppm |
| PHO-Gehalt | 4 ppm | 4 ppm | 1 ppm |
| ETF-Gehalt | 50 ppm | 200 ppm | 50 ppm |
| Molverhältnis ETF:PHO | 8.1 | 32.6 | 32.6 |
| Viskosität | 30 mPa.s | 30 mPa.s | 33 mPa.s |
| NCO-Gehalt | 29.6 Gew.-% | 29.6 Gew.-% | 29.5 Gew.-% |

### Beispiel 3: Lagerungsversuche

Die aus Beispiel 2 erhaltenen Proben wurden einer simulierten beschleunigten Lagerung durch Erhitzen auf 100 °C für drei Tage unterworfen. Anschließend wurden Viskosität und NCO-Gehalt erneut bestimmt.

| Beispiel Nr. | 3-1 (Vergleich) | 3-2 (Vergleich) | 3-3 (erfindungsgemäß) |
|---|---|---|---|
| Probe aus Beispiel | 2-1 | 2-2 | 2-3 |
| Viskosität | 82 mPa.s | 65 mPa.s | 58 mPa.s |
| NCO-Gehalt | 26.9 Gew.-% | 27.7 Gew.-% | 28.5 Gew.-% |

Unter den drastischen Lagerungsbedingungen von 100 °C für drei Tage zeigte sich, dass selbst bei gleich hohem ETF:PHO-Verhältnis die Stabilität der erfindungsgemäß hergestellten Zusammensetzung - erkennbar an der geringsten Zunahme der Viskosität und der geringsten Abnahme des NCO-Gehalts - am besten ist.

## Patentansprüche

1. Verfahren zur Herstellung einer Polycarbodiimide umfassenden Zusammensetzung, umfassend den Schritt der Umsetzung einer Reaktionsmischung, welche ein aromatisches Polyisocyanat und einen Carbodiimidisierungs-Katalysator umfasst,
**dadurch gekennzeichnet, dass**
vor der Umsetzung das aromatische Polyisocyanat bei einer Temperatur von ≥ 80 °C bis ≤ 150 °C und einem Druck von ≥ 1 mbar bis ≤ 500 mbar mittels Durchleiten eines Inertgases behandelt wird
und/oder
während der Umsetzung die Reaktionsmischung bei einer Temperatur von ≥ 80 °C bis ≤ 150 °C und einem Druck von ≥ 1 mbar bis ≤ 500 mbar mittels Durchleiten eines Inertgases behandelt wird,
wobei weiterhin in der Reaktionsmischung der Gehalt an hydrolysierbarem Chlor ≤ 10 ppm beträgt.

2. Verfahren gemäß Anspruch 1, wobei in der Reaktionsmischung der Gehalt des Carbodiimidisierungs-Katalysators ≤ 5 ppm, bevorzugt < 3,5 ppm, besonders bevorzugt < 3 ppm beträgt.

3. Verfahren gemäß Anspruch 1 oder 2, wobei das aromatische Polyisocyanat Diphenylmethandiisocyanat ist.

4. Verfahren gemäß einem der Ansprüche 1 bis 3, wobei der Carbodiimidisierungs-Katalysator ein Phospholinoxid ist.

5. Verfahren gemäß einem der Ansprüche 1 bis 4, wobei die Umsetzung in Abwesenheit eines Säurefängers erfolgt.

6. Verfahren gemäß einem der Ansprüche 1 bis 5, wobei nach dem Ende der Umsetzung der Reaktionsmischung zu dem hierdurch erhaltenen Produkt ein Stopper hinzugefügt wird.

7. Verfahren gemäß Anspruch 6, wobei der Trifluormethansulfonsäureester in einer Menge von ≥ 10 ppm bis ≤ 100 ppm hinzugefügt wird.

8. Verfahren gemäß einem der Ansprüche 1 bis 7, wobei nach dem Ende der Umsetzung der Reaktionsmischung zu dem hierdurch erhaltenen Produkt kein Säurefänger hinzugefügt wird.

9. Verfahren gemäß einem der Ansprüche 1 bis 8, wobei nach dem Ende der Umsetzung der Reaktionsmischung zu dem hierdurch erhaltenen Produkt ein Polyisocyanat hinzugefügt wird.

10. Verfahren gemäß einem der Ansprüche 1 bis 9, wobei nach dem Ende der Umsetzung der Reaktionsmischung das hierdurch erhaltene Produkt mit einem Polyol zur Reaktion gebracht wird.

## Claims

1. Process for producing a composition comprising polycarbodiimides, which comprises the step of reacting a reaction mixture comprising an aromatic polyisocyanate and a carbodiimidization catalyst, **characterized in that**
before the reaction, the aromatic polyisocyanate is treated at a temperature of from ≥ 80°C to ≤ 150°C and a pressure of from ≥ 1 mbar to ≤ 500 mbar by means of passing an inert gas through it,
and/or
during the reaction, the reaction mixture is treated at a temperature of from ≥ 80°C to ≤ 150°C and a pressure of from ≥ 1 mbar to ≤ 500 mbar by means of passing an inert gas through it,
with, in addition, the content of hydrolyzable chlorine in the reaction mixture being ≤ 10 ppm.

2. Process according to Claim 1, wherein the content of the carbodiimidization catalyst in the reaction mixture is ≤ 5 ppm, preferably < 3.5 ppm, particularly preferably < 3 ppm.

3. Process according to Claim 1 or 2, wherein the aromatic polyisocyanate is diphenylmethane diisocyanate.

4. Process according to any of Claims 1 to 3, wherein the carbodiimidization catalyst is a phospholine oxide.

5. Process according to any of Claims 1 to 4, wherein the reaction is carried out in the absence of an acid scavenger.

6. Process according to any of Claims 1 to 5, wherein a stopper is added to the product obtained after the end of the reaction of the reaction mixture.

7. Process according to Claim 6, wherein the trifluoromethanesulfonic ester is added in an amount of from ≥ 10 ppm to ≤ 100 ppm.

8. Process according to any of Claims 1 to 7, wherein no acid scavenger is added to the product obtained after the end of the reaction of the reaction mixture.

9. Process according to any of Claims 1 to 8, wherein a polyisocyanate is added to the product obtained after the end of the reaction of the reaction mixture.

10. Process according to any of Claims 1 to 9, wherein the product obtained from the reaction of the reaction mixture is reacted with a polyol after the end of the reaction.

## Revendications

1. Procédé pour la préparation d'une composition comprenant du polycarbodiimide, comprenant l'étape de transformation d'un mélange réactionnel, qui comprend un polyisocyanate aromatique et un catalyseur de carbodiimidisation, **caractérisé en ce**
**qu'**avant la transformation, le polyisocyanate aromatique est traité à une température de ≥ 80°C à ≤ 150°C et à une pression de ≥ 1 mbar à ≤ 500 mbars par passage d'un gaz inerte
et/ou
pendant la transformation, le mélange réactionnel est traité à une température de ≥ 80°C à ≤ 150°C et à une pression de ≥ 1 mbar à ≤ 500 mbars par passage d'un gaz inerte,
la teneur en chlore hydrolysable dans le mélange réactionnel étant en outre ≤ 10 ppm.

2. Procédé selon la revendication 1, la teneur en catalyseur de carbodiimidisation dans le mélange réactionnel étant ≤ 5 ppm, de préférence < 3,5 ppm, de manière particulièrement préférée < 3 ppm.

3. Procédé selon la revendication 1 ou 2, le polyisocyanate aromatique étant le diisocyanate de diphénylméthane.

4. Procédé selon l'une quelconque des revendications 1 à 3, le catalyseur de carbodiimidisation étant un oxyde de phospholine.

5. Procédé selon l'une quelconque des revendications 1 à 4, la transformation ayant lieu en l'absence d'un piège d'acide.

6. Procédé selon l'une quelconque des revendications 1 à 5, un agent d'arrêt étant ajouté après la fin de la transformation du mélange réactionnel au produit obtenu par cette transformation.

7. Procédé selon la revendication 6, l'ester de l'acide trifluorométhanesulfonique étant ajouté en une quantité de ≥ 10 ppm à ≤ 100 ppm.

8. Procédé selon l'une quelconque des revendications 1 à 7, aucun piège d'acide n'étant ajouté après la fin de la transformation du mélange réactionnel au produit obtenu par cette transformation.

9. Procédé selon l'une quelconque des revendications 1 à 8, un polyisocyanate étant ajouté après la fin de la transformation du mélange réactionnel au produit obtenu par cette transformation.

10. Procédé selon l'une quelconque des revendications 1 à 9, le produit obtenu par la transformation étant mis à réagir avec un polyol après la fin de la transformation du mélange réactionnel.
